# EUROPEAN PATENT APPLICATION

(11) **EP 4 850 683 A1**
(43) Date of publication of application: **09.12.2026**
(21) Application number: 25181482.8
(22) Date of filing: 06.06.2025
(51) Int. Cl.: A61K 38/57, A61K 31/506, A61K 31/553, A61K 33/243, A61P 35/00

(54) **PHARMACEUTICAL COMPOUND FOR TREATMENT OF CANCER**

(71) Applicant: Medizinische Hochschule Hannover, 30625 Hannover (DE)
(72) Inventor: Janciauskiene Wallmark, Sabina Marija, 30625 Hannover (DE); Sivaraman, Kokilavani, 30625 Hannover (DE)
(74) Representative: Taruttis, Stefan Georg

(57) **Abstract**

The invention provides a compound for use in the treatment of cancer, which compound comprises or consists of a peptide, preferably, the compound is for use in the treatment of cancer in combination with the use of at least one apoptosis-inducing pharmaceutical.

## Description

The present invention relates to a compound for use in the treatment of cancer, especially to a combination of the compound with an anti-cancer pharmaceutical. Accordingly, the invention provides a compound for use in the treatment of cancer, preferably to a combination of the compound with an anti-cancer pharmaceutical, wherein the compound may be for administration to a patient separate from administration of the anti-cancer pharmaceutical, e.g. prior to or subsequent to administration of the anti-cancer pharmaceutical, or the compound may be for administration concurrent with administration of the anti-cancer agent, e.g. in one composition or as separate compositions, each composition comprising e.g. a pharmaceutically acceptable vehicle, e.g. solvent, diluent, preferably aqueous. The compound has the advantage of being an active agent for use in the treatment of cancer, which cancer may even be resistant against an apoptosis-inducing pharmaceutic, e.g. the cancer may be a relapse from treatment using an apoptosis-inducing pharmaceutic.

Accordingly, the compound may be for use in the treatment of cancer subsequent to treatment of the patient using an apoptosis-inducing pharmaceutic.

### State of the art

Sivaraman et al., Journal of Leukocyte Biology 58-70 (2023) in an animal model of bacterial lung infection using aerosolized bacterial LPS analysed the effect of i.p. administration of alpha-1-antitrypsin (AAT) or derivatives of fragments of AAT.

Blaurock et al., Mediators of Inflammation, Vol. 2016, Article ID 6129437 describe a C-terminal fragment of AAT as a diagnostic marker for infectious and non-infectious etiologies of systemic inflammatory response syndrome.

Bigalke et al., Scientific Reports 12:3844 (2022) describe analysis of C-terminal peptides of alpha-1-antitrypsin in blood in sepsis-afflicted patients.

Zelvyte et al., Cancer Cell International 4:7 (2004) found that cultivation of lung cancer HCC cells in medium conditioned with polymorphonuclear neutrophils (PMN) showed decreased proliferation and IL-8 release, but increased invasiveness, whereas additional presence of AAT or its C-terminal 36 fragment abolished the effect of PMN conditioned medium but did not alter its effect on cell proliferation or IL-8 and VEGF release.

Tammen et al., Methods 240, 7-13 (2025) describe quantitative mass spectrometry of the C-terminal 36 amino acid peptides derived from alpha-1-antitrypsin in plasma.

Scherr et al., describe that proteases of Staphylococcus aureus cleave AAT, generating a C-terminal fragment of 36 amino acids (C36), as well as C-terminal fragments C42 and C40.

Janciauskiene et al., Front Oncol. 11:622076 (2021) analyse the uptake of AAT by cancer cells and describe an anti-apoptotic effect of AAT when treating cancer cells with Staurosporin.

For treatment of cancer, apoptosis-inducing pharmaceutics are known, e.g. Cisplatin, Adiposide.

### Object of the invention

It is an object of the invention to provide a compound suitable for use in the treatment of cancer, wherein preferably the compound is suitable for treatment of cancer refractory to apoptosis-inducing pharmaceutics, and/or wherein the compound allows to reduce the dosage of an apoptosis-inducing pharmaceutic, e.g. in a combination of the apoptosis-inducing pharmaceutical with the compound.

### Object of the invention

The invention achieves the object by providing a compound for use in the treatment of cancer, which compound comprises or consists of a peptide of amino acids No. 1-42 to 7-42, e.g. amino acids No. 2-42, 3-42, 4-42, 5-42, 6-42, of amino acid sequence LEAIPMSIPPEVKFNKPFVFLMIEQNTKSPLFMG KVVNPTQK (SEQ ID NO: 1), which peptide e.g. comprises or consists at least of SEQ ID NO: 1, herein also referred to as C42, and/or comprises or consists at least of a peptide of amino acid sequence SIPPEVKFNKPFVFLMIEQNTKSPLFMGKVVNPTQK (SEQ ID NO: 2), herein also referred to as C36. Generally, amino acid sequences are described from N-terminus to C-terminus. Preferably, the compound is for use in the treatment of cancer in combination with the use of at least one apoptosis-inducing pharmaceutical.

Optionally, the compound is bound to a pharmaceutically acceptable carrier, such as a nanoparticle. Suitable nanoparticles include metal nanoparticles, e.g. gold nanoparticles (AuNPs); metal oxide nanoparticles, e.g. nanoparticles of iron oxide (Fe₃O₄), zinc oxide (ZnO), or cerium oxide (CeO₂); or synthetic polymer-based nanoparticles, e.g. nanoparticles of poly(lactic-co-glycolic acid) (PLGA), polyethylene glycol-polylactic acid (PEG-PLA) copolymers, or polymethyl methacrylate (PMMA). These carriers can improve the solubility, stability, bioavailability, and targeted delivery of the compound.

Optionally, the compound can be associated with a pharmaceutically acceptable carrier, for example, encapsulated in liposomes such as unilamellar or multilamellar phospholipid vesicles. Liposomes may optionally be modified with polyethylene glycol (PEG) or surface ligands to enhance pharmacokinetic properties and targeting specificity. The compound can be included in a pharmaceutical formulation for injection, especially for local delivery to a tumour site by injection, as a spray for forming an aerosol.

The compound is especially suitable for use in the treatment of cancer that is resistant to at least one apoptosis-inducing pharmaceutical agent. Such resistance may arise from genetic mutations, dysregulation of pro-apoptotic or anti-apoptotic signaling pathways (e.g., overexpression of Bcl-2, loss of p53 function, or upregulation of IAPs), or from the activation of survival pathways such as PI3K/AKT or MAPK. In such apoptosis-resistant tumors, conventional chemotherapeutic agents or targeted therapeutics that rely on the induction of programmed cell death (apoptosis) often fail to achieve adequate clinical efficacy. The compound described herein exerts its anti-cancer activity through a mechanism that is at least partially independent of the canonical apoptotic pathways and may involve alternative forms of regulated cell death, such as ferroptosis, necroptosis, or lysosomal cell death, or through the disruption of essential tumor cell metabolism or survival signaling. As a result, the compound provides a novel therapeutic option for malignancies that are refractory to current pro-apoptotic treatments, including but not limited to platinum-based chemotherapeutics, BH3 mimetics, TRAIL-receptor agonists, or death receptor-targeting agents.

It has been found that the compound, e.g. as a single active agent for use in the treatment of cancer, induces the formation of large vacuoles in cancer cells, which formation is believed to be associated with disruption of the endoplasmatic recticulum. According to present analyses, the compound does not induce fragmentation of cellular DNA, indicating that the compound alone does not induce apoptosis. Further, it has been found that the compound is especially suitable for use in the treatment of cancer that has a metabolism highly dependent on iron. Preferably, e.g. when used as a single active agent in the treatment of cancer, the cancer has an expression of TfR1 (CD71) higher than in non-cancerous cells, e.g. an expression of TfR1 higher by at least 30%, by at least 50% or by at least 100%, e.g. as determined as expression of the TFRC gene. Therein, non-cancerous cells preferably are cells of the same tissue or cell type as the cancer being treated. This allows for direct comparative assessment of selectivity and therapeutic window in vitro and/or in vivo. Accordingly, when the compound is administered as a single active pharmaceutical agent in the treatment of cancer, or in combination with an apoptosis-inducing pharmaceutical agent (e.g., a chemotherapeutic drug or targeted apoptosis modulator), it is believed to induce a mechanism of cell death that differs fundamentally from classical apoptosis. This mechanism is herein referred to as ferroptosis, a regulated form of non-apoptotic cell death characterized by iron-dependent accumulation of lipid peroxides and oxidative stress. More specifically, ferroptosis involves elevated intracellular levels of ferrous iron (Fe²⁺), increased generation of reactive oxygen species (ROS), and peroxidation of polyunsaturated fatty acid residues in cellular membranes, ultimately leading to loss of membrane integrity and cell death.

Importantly, the compound induces ferroptosis preferentially in cancer cells as compared to non-cancerous cells of the same tissue origin, suggesting a degree of therapeutic selectivity. This selectivity may be attributed to metabolic or redox imbalances commonly observed in cancer cells, such as dysregulated iron metabolism, elevated basal ROS levels, or altered expression of antioxidant defences including glutathione peroxidase 4 (GPX4) or SLC7A11 (xCT). As a result, cancer cells exhibit heightened sensitivity to ferroptosis inducers.

Preferably, the cancer to be treated according to the present invention is one that is known to exhibit resistance to apoptosis-based therapies or that possesses metabolic features predisposing it to ferroptosis susceptibility. Non-limiting examples of such cancers include non-small cell lung cancer (NSCLC), triple-negative breast cancer (TNBC), hepatocellular carcinoma (HCC), glioblastoma (GBM), pancreatic ductal adenocarcinoma (PDAC), ovarian carcinoma, and malignant melanoma. These cancers are characterized by poor prognosis and limited response to conventional therapies, highlighting the urgent need for therapeutic agents acting via non-apoptotic mechanisms such as ferroptosis.

The compound described herein may be administered systemically or locally, optionally formulated with a pharmaceutically acceptable carrier such as liposomes, metal or metal oxide nanoparticles, or biodegradable synthetic polymers, as disclosed elsewhere in this document. Co-administration with apoptosis-inducing agents may further enhance efficacy through combinatorial or synergistic interactions between ferroptotic and apoptotic pathways.

Accordingly, the compound is suitable for use in the treatment of cancer that is refractory to treatment by a apoptosis-inducing pharmaceutical as the only active agent, and the compound is suitable for use in the treatment of cancer in a patient subsequent to therapy by an apoptosis-inducing pharmaceutic, e.g. for treatment of patients having a relapse, especially a relapse after treatment by a apoptosis-inducing pharmaceutic.

Further, it has been found that in use in the treatment of cancer, the compound enhances the efficacy of apoptosis-inducing pharmaceutics and that the compound in combination with an apoptosis-inducing pharmaceutical is suitable for use in the treatment of cancer, both for cancer susceptible to an apoptosis-inducing pharmaceutical and for cancer refractory to an apoptosis-inducing pharmaceutic.

Herein, a preferred apoptosis-inducing pharmaceutical agent is selected from Cisplatin, Adavosertib (Adavoside), Etoposide, Staurosporine, Osimertinib, Doxorubicin, Paclitaxel, Vincristine, Bortezomib, Venetoclax, Sorafenib, or a combination of at least two of these agents. These agents include DNA-damaging chemotherapeutics, topoisomerase inhibitors, microtubule stabilizers, proteasome inhibitors, tyrosine kinase inhibitors, and Bcl-2 antagonists, each known to induce apoptotic cell death through distinct pathways. These agents are commonly used in clinical oncology to initiate apoptosis in cancer cells via various mechanisms, including DNA damage, topoisomerase inhibition, or kinase inhibition. However, resistance to apoptosis remains a significant limitation in the clinical utility of such agents, particularly in aggressive and relapsed tumors.

A particularly preferred apoptosis-inducing pharmaceutical agent for use in combination with the compound described herein is Osimertinib, a third-generation epidermal growth factor receptor (EGFR) tyrosine kinase inhibitor (TKI). Osimertinib is suggested to influence intracellular iron homeostasis and redox metabolism, including modulation of ferritin expression and iron uptake pathways. These metabolic alterations are believed to potentiate the ferroptosis-inducing activity of the compound by increasing intracellular labile Fe²⁺ pools and thereby facilitating lipid peroxidation. Thus, Osimertinib may act synergistically or additively with the compound to enhance cancer cell death via ferroptosis, particularly in tumors with EGFR mutations or elevated iron sensitivity.

When used in combination with the compound, the apoptosis-inducing pharmaceutical agent may be administered at a reduced dosage compared to its dosage used in standard monotherapy regimen. For example, the dosage of the apoptosis-inducing agent may be reduced to approximately 70%, 60%, 50%, 40%, or even 30% of the conventional therapeutic dose used when administered as a single active agent. This reduction is applicable whether the apoptosis-inducing pharmaceutical is used alone or in combination with one or more additional such agents.

An important advantage of combining the compound with one or more apoptosis-inducing pharmaceutical agents is the potential for a significant reduction in the dose-dependent adverse effects typically associated with such agents. Many clinically used apoptosis inducers, while effective, are limited by their toxicity profiles. For example, Cisplatin is well-documented to cause dose-limiting nephrotoxicity, ototoxicity, and neurotoxicity; Etoposide frequently leads to myelosuppression, including neutropenia and thrombocytopenia; and agents such as Doxorubicin are associated with cumulative cardiotoxicity. Other commonly used agents, including Paclitaxel and Staurosporine, can induce severe gastrointestinal disturbances, peripheral neuropathy, or hepatotoxicity.

By enabling the induction of an alternative, non-apoptotic form of regulated cell death-specifically ferroptosis-the compound described herein facilitates the use of reduced doses of the apoptosis-inducing pharmaceuticals while maintaining or enhancing overall anti-tumor efficacy. This reduction in required dose may be achieved, for instance, by lowering the standard monotherapy dose of the apoptosis-inducing agent to approximately 70%, 60%, 50%, 40%, or even 30%, as discussed elsewhere in this disclosure.

The resulting reduction in systemic toxicity is anticipated to enhance patient tolerability, minimize treatment interruptions, and allow for prolonged administration cycles, particularly in patients with comorbidities or those previously exposed to high cumulative doses of chemotherapeutics. Moreover, the improved safety profile may increase the therapeutic index of the combination regimen, enabling more effective tumor control at lower toxicity risk.

This combinatorial approach is particularly advantageous in the treatment of aggressive or therapy-resistant cancers where dose intensification of standard therapies is not feasible due to intolerable side effects. Thus, the compound provides a dual benefit in enhancing efficacy while mitigating adverse events typically associated with apoptosis-based anticancer drugs.

An important advantage of combining the compound with one or more apoptosis-inducing pharmaceutical agents is the potential for a significant reduction in the dose-dependent adverse effects typically associated with such agents. Many clinically used apoptosis inducers, while effective, are limited by their toxicity profiles. For example, Cisplatin is well-documented to cause dose-limiting nephrotoxicity, ototoxicity, and neurotoxicity; Etoposide frequently leads to myelosuppression, including neutropenia and thrombocytopenia; and agents such as Doxorubicin are associated with cumulative cardiotoxicity. Other commonly used agents, including Paclitaxel, can induce severe gastrointestinal disturbances, peripheral neuropathy, or hepatotoxicity.

By enabling the induction of an alternative, non-apoptotic form of regulated cell death-specifically ferroptosis-the compound described herein facilitates the use of reduced doses of the apoptosis-inducing pharmaceuticals while maintaining or enhancing overall anti-tumor efficacy. This reduction in required dose may be achieved, for instance, by lowering the standard monotherapy dose of the apoptosis-inducing agent to approximately 70%, 60%, 50%, 40%, or even 30%, as discussed elsewhere in this disclosure.

The resulting reduction in systemic toxicity is anticipated to enhance patient tolerability, minimize treatment interruptions, and allow for prolonged administration cycles, particularly in patients with comorbidities or those previously exposed to high cumulative doses of chemotherapeutics. Moreover, the improved safety profile may increase the therapeutic index of the combination regimen, enabling more effective tumor control at lower toxicity risk. This combinatorial approach is particularly advantageous in the treatment of aggressive or therapy-resistant cancers where dose intensification of standard therapies is not feasible due to intolerable side effects. Thus, the compound provides a dual benefit in enhancing efficacy while mitigating adverse events typically associated with apoptosis-based anticancer drugs.

Moreover, the combination strategy disclosed herein enables the simultaneous targeting of multiple, mechanistically distinct cell death pathways-namely apoptosis and ferroptosis. This dual-pathway approach offers a significant therapeutic advantage by addressing tumor heterogeneity and overcoming adaptive resistance mechanisms that frequently limit the long-term efficacy of monotherapies. Many cancers develop resistance to apoptosis-inducing agents through mutations in key regulators such as TP53, BCL-2 family proteins, or caspases, or via alterations in death receptor signaling. By incorporating a compound that induces ferroptosis, which is mechanistically independent of caspase activation and mitochondrial outer membrane permeabilization, the treatment regimen circumvents these common resistance routes.

Importantly, ferroptosis is characterized by iron-dependent lipid peroxidation and is driven by metabolic and oxidative vulnerabilities that are often enhanced in cancer cells, such as dysregulated iron metabolism, elevated basal ROS levels, or defective antioxidant systems (e.g., downregulation of GPX4 or SLC7A11). As such, the combination of a ferroptosis-inducing compound with an apoptosis-inducing pharmaceutical may produce additive or synergistic anti-tumor effects while minimizing the risk of resistance development and tumor escape.

This combinatorial strategy is particularly advantageous in the treatment of therapy-resistant malignancies, including but not limited to non-small cell lung cancer (NSCLC), triple-negative breast cancer (TNBC), hepatocellular carcinoma (HCC), glioblastoma (GBM), pancreatic ductal adenocarcinoma (PDAC), ovarian carcinoma, and malignant melanoma. These cancer types are frequently associated with poor prognosis, limited responsiveness to conventional chemotherapy or targeted therapies, and high recurrence rates. Therefore, the disclosed combination approach represents a promising therapeutic avenue for enhancing treatment efficacy and clinical outcome in cancers that are refractory to standard-of-care pro-apoptotic regimens.

Optionally, the compound is for use in combination with iron ions, e.g. Fe2+, as it was shown that in ferroptosis iron ions, e.g. Fe2+, accumulate in cancer cells.

Preferably, the compound is the product of chemical synthesis of its amino acid sequence.

As an advantage, it was found that the peptide compound of the invention is not toxic to healthy cells.

In the in vitro examples, it was found that some cancer cells when cultivated to high density, e.g. up to confluence, have low expression of TFRC, indicating a low iron-dependent metabolism, and show low sensitivity to presence of peptide C36 or peptide C42. This low sensitivity for cancer cells in vitro cultivated to high cell density, and low expression of TFRC, is currently believed to be an in vitro artefact. In contrast, when adding peptide C36 or peptide C42 to culture medium at low density of cancer cells, the cancer cells showed high sensitivity, i.e. high killing rates due to peptide C36 or peptide C42, despite low expression of TFRC. Therefore, the compound, especially peptide C36 and/or peptide C42, are suitable for for use in the treatment of cancer, wherein the cancer shows high expression of TFRC or low expression of TFRC, and preferably in combination with administration of an apoptosis-inducing pharmaceutical. Generally, expression of TFRC is preferably determined in relation to a household gene, e.g. in relation to expression of POLR2A.

The invention is now described in greater detail by way of examples and with reference to the figures, which show in
- Fig. 1 the proportion of H1563 and of H1299 cancer cells associated with FITC-labelled C36 peptide,
- Fig. 2A - 2F confocal microscopy images of H1563 and of H1299 cancer cells,
- Fig. 2G - 2H ... images of H1563 and of H1299 cancer cells,
- Fig. 3A - 3D microscopy images of H1299 and H1563 cancer cells,
- Fig. 4A - 4F expression levels of genes related to metabolism in H1563 cancer cells,
- Fig. 5A, 5B microscopy images of H1563 cancer cells with staining for ER,
- Fig. 5C, 5D light microscopy images of H1563 cancer cells,
- Fig. 5E an overlay of Fig. 5A with Fig. 5C,
- Fig. 5F an overlay of Fig. 5B with Fig. 5D,
- Fig. 6A expression levels of the ATF6 gene in H1563 cancer cells,
- Fig. 6B expression levels of the DDIT3 gene in H1563 cancer cells,
- Fig. 6C expression levels of the HSPA5 gene in H1563 cancer cells,
- Fig. 6D expression levels of the EIF2AK3 gene in H1563 cancer cells,
- Fig. 6E expression levels of the ERN gene in H1563 cancer cells,
- Fig. 7A, 7B microscopy images of H1299 cancer cells with staining for ER,
- Fig. 7C, 7D light microscopy images of H1299 cancer cells,
- Fig. 7E an overlay of Fig. 7A with Fig. 7C,
- Fig. 7F an overlay of Fig. 7B with Fig. 7D,
- Fig. 8A expression levels of the XBP1 gene in H1299 cancer cells,

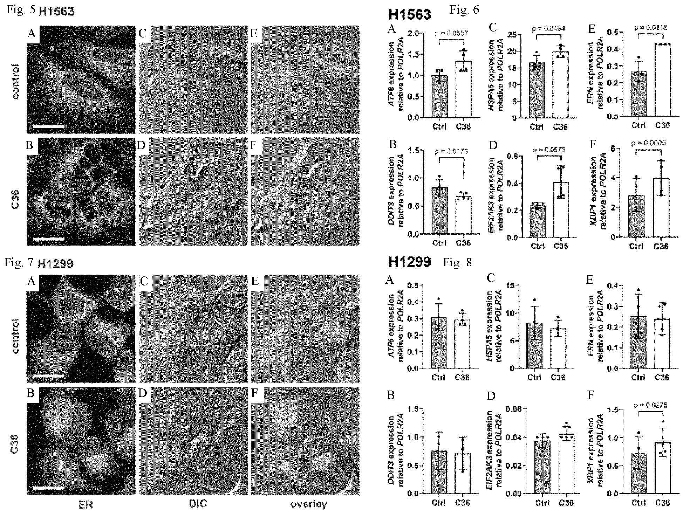

- Fig. 8A expression levels of the ATF6 gene in H1299 cancer cells,
- Fig. 8B expression levels of the DDIT3 gene in H1299 cancer cells,
- Fig. 8C expression levels of the HSPA5 gene in H1299 cancer cells,
- Fig. 8D expression levels of the EIF2AK3 gene in H1299 cancer cells,
- Fig. 8E expression levels of the ERN gene in H1299 cancer cells,
- Fig. 8A expression levels of the XBP1 gene in H1299 cancer cells,

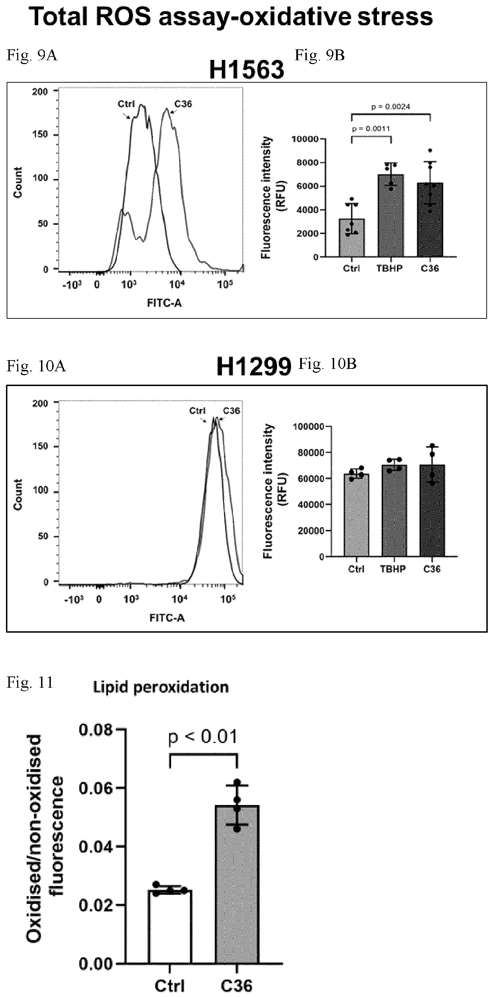

- Fig. 9A a FACS measurement for ROS for H1563 cancer cells without (Ctrl) and with C36 (C36),
- Fig. 9B the result of ROS measurement for H1563 cancer cells of Fig. 9A and a positive control (TBHP),
- Fig. 10A shows a FACS measurement for ROS for H1299 cancer cells without (Ctrl) and with C36 (C36),
- Fig. 10B the result of ROS measurement for H1299 cancer cells of Fig. 9A and a positive control (TBHP),
- Fig. 11 FACS measurement results indicating peroxidation of lipids in H1563 cancer cells,

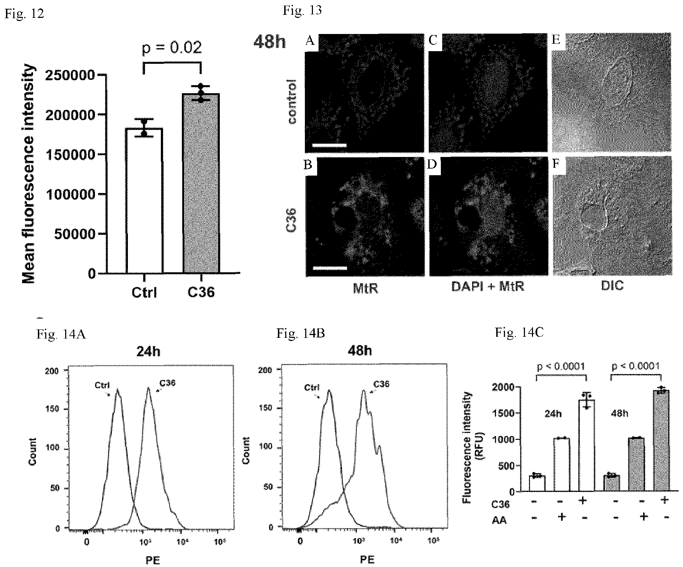

- Fig. 12 FACS results for the mitochondrial membrane potential for H1563 cells,
- Fig. 13A, 13B microscopy images of H1563 cells with staining for mitochondria,
- Fig. 13C, 13D microscopy images of H1563 cells with staining for mitochondria and nuclei,
- Fig. 13E, 13F microscopy images of H1563 cells,
- Fig. 14A, 14B FACS measurements for mitochondrial reactive oxygen species (ROS) levels,
- Fig. 14C results of ROS measurements of Fig. 14A, 14B,

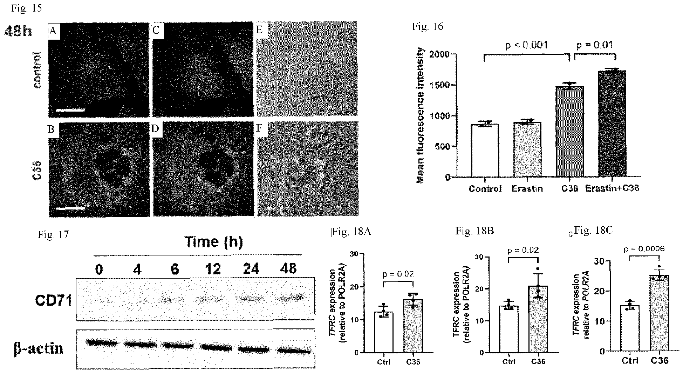

- Fig. 15A, 15B microscopy images of H1563 cells with staining for Fe2+,
- Fig. 15C, 15D microscopy images of H1563 cells with staining for Fe2+ and counterstaining for nuclei,
- Fig. 15E, 15F microscopy images of H1563 cells,
- Fig. 16 FACS results for intracellular Fe²⁺,
- Fig. 17 Western blots for TFRC,
- Fig. 18 qRT-PCR of TFRC encoding mRNA,

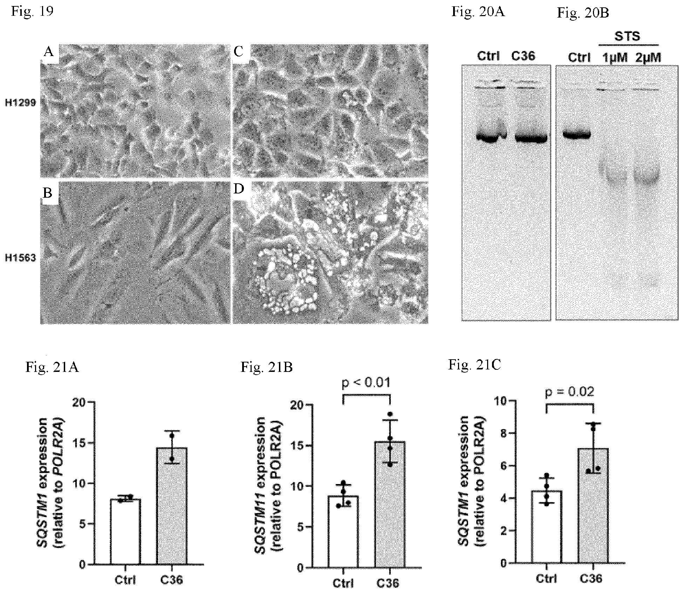

- Fig. 19A, 19C show light microscopy images of H1299 cells,
- Fig. 19B, 19D show light microscopy images of H1563 cells
- Fig. 20A, 20B total DNA from H1563 cells,
- Fig. 21 results of qRT-PCR analysis for SQSTM1 expression levels in H1563 cells,

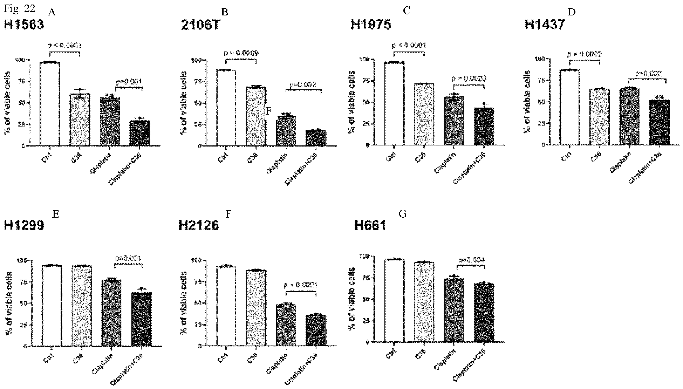

- Fig. 22A for H1563, 22B for 2106T cells, 22C for H1975 cells, 22D for H1437 cells, 22E for H1299 cells, 22F for H2126 cells, 22G for H661 cells, the survival in contact with C36 or with the combination of C36 with cisplatin,

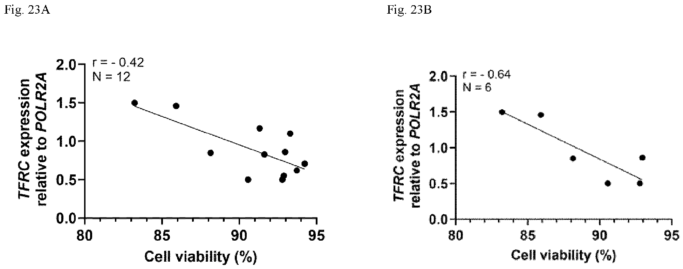

- Fig. 23A, 23B cancer cell viability in dependence on TFRC expression,

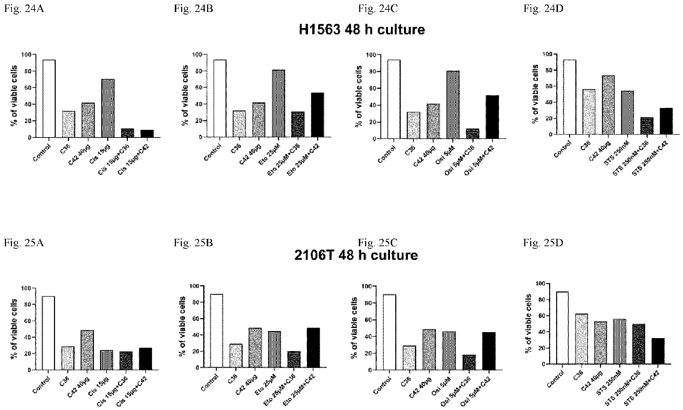

- Fig. 24 cancer cell viability for different concentrations of cisplatin in combination with peptide C36,
- Fig. 25 the viability of cancer cells when treated with peptide C36 in combination with different apoptosis-inducing pharmaceuticals,

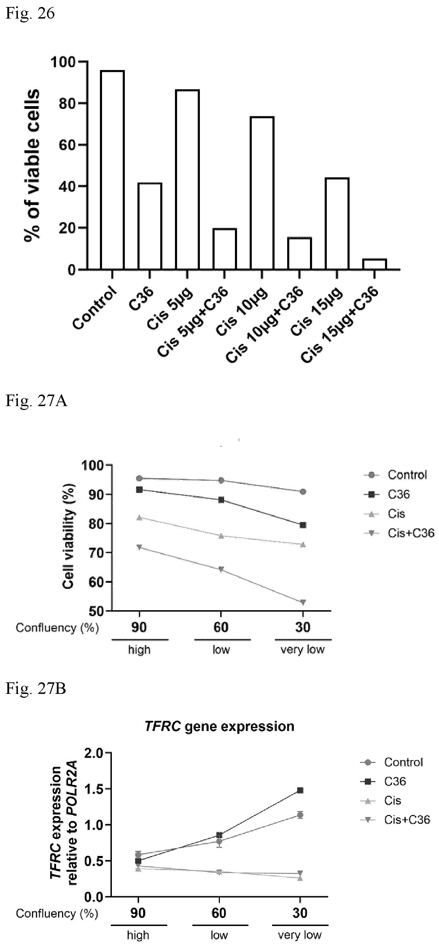

- Fig. 26 viability of cancer cells when treated with peptide C36 in combination with different concentrations with Cisplatin, and
- Fig. 27A cancer cell viability at different cell densities in culture in presence of peptide C36 and for peptide C36 in combination with Cisplatin,
- Fig. 27B expression levels of TFRC in H1299 cancer cells at different cell densities in culture in presence of peptide C36 and for peptide C36 in combination with Cisplatin.

Generally, in each Fig. the microscopy images are at the same magnification.

### Example 1: In vitro killing of cancer cells by peptide C36

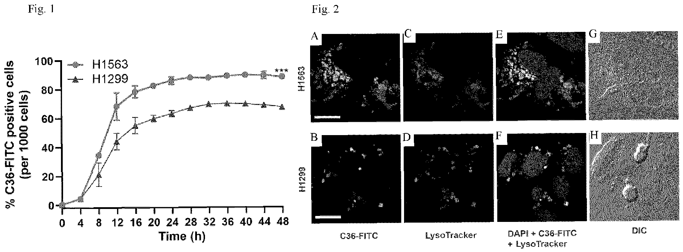

As a representative of the peptide of the invention, the peptide of SEQ ID NO: 2 (C36) was synthesized and contacted with cancer cell lines H1563 or H1299 cultivated in RPMI-1640 medium with 10% bovine calf serum by adding the peptide C36 to a final concentration in the culture medium. For imaging, the peptide C36 was labelled with a green, fluorescent dye (FITC). The association of the peptide C36 was determined for the static culture every 4 hours using a Biotek cytation imager. Fig. 1 shows the percentage of fluorescently labelled cells, indicating association of the peptide C36 with cells. The time-course shows that the peptide C36 became associated with the cancer cells during the cultivation within approx. 16 to 20 hours to at least 50 % of H1299 and 80 % of H1563 cells.

Figs. 2A, 2C, 2E and 2G show the same H1563 cells in each analysis, and Figs. 2B, 2D, 2F and 2H show the same H1299 cells. Therein, Figs. 2A and 2B (scale bar 20 µm) show the localisation of the FITC-labelled C36 (C36-FITC, Fluorescein Isothiocyanate - green fluorescent dye in biological and biochemical research), and Figs. 2C and 2D show lysotracker (red), Figs. 2E and 2F show the localisation of DAPI (blue), of the labelled peptide C36 (C36-FITC) and of LysoTracker, showing the peptide C36 was localized in lysosomes (yellow). Figs. 2G and 2H show the shape and edges of the cells (DIC stands for Differential Interference Contrast).

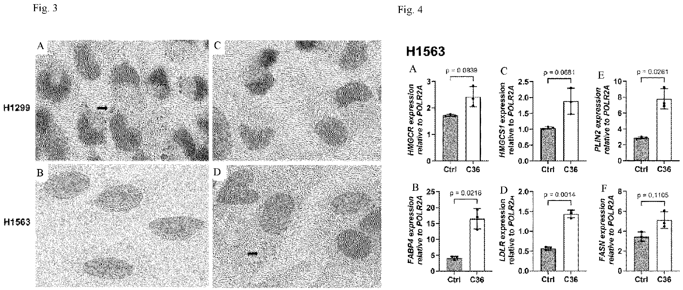

Figs. 3A and 3C show cultivated H1299 cells and Figs. 3B and 3D show H1563 cells, each with oil red staining for intracellular lipid droplets, in Figs. 3A-3B in 1640 RPMI supplemented with 10% bovine calf serum without added C36 and in Figs. 3C-3D in 1640 RPMI containing 10% bovine calf serum with 40 µg/mL C36, without label, after static cultivation at 37°C for 48h. Figs. 3A, 3C show that H1299 cells had lipid droplets at baseline and did not change in response to C36 peptide, Figs. 3B, 3D show that H1563 has little to no droplets at the baseline and a markedly increased lipid droplet formation (intracellular lipid accumulation) after treatment, indicating change in lipid metabolism due to presence of C36.

Fig. 4 shows results for H1563 cells of quantitative PCR analyses, normalized to expression of POLR2A, in Fig. 4A for 3-hydroxy-3-methyl-glutaryl-coenzyme A reductase (HMGCR) expression, in Fig. 4B for Fatty Acid Binding Protein 4 (FABP4) expression, in Fig. 4C for 3-Hydroxy-3-Methylglutaryl-CoA Synthase 1 (HMGCS) expression, in Fig. 4D for Low Density Lipoprotein Receptor (LDLR) expression, in Fig. 4E for Perilipin 2 (PLIN2) expression, and in Fig. 2F for Fatty acid synthase (FASN) expression, indicating an increase in lipid metabolism related genes due to presence of C36 (right hand columns) compared to medium only (Ctrl, left hand columns).

Figs. 5 to 6 show the effects of treatment by 40 µg/mL C36 (diluted in water) on cultivated H1563 cancer cells, Figs. 7 to 8 on H1299 cancer cells, in Fig. 5A, 5C, 5E, resp. Fig. 7A, 7C, 7E for cultivation in medium (control), in Fig. 5B, 5D, 5F, resp. Fig. 7B, 7D, 7F for cultivation in medium with added C36, scale bar 20 µm. During cultivation, H1563 and H1299 cancer cell lines were treated with C36 for 48 h and the endoplasmic reticulum (ER) was stained using LipoFluor-ER2 (green). Controls in Fig. 5A (control) and Fig. 7A (control) show the staining of the ER for cultivation in medium only, Fig. 5D and Fig. 7D show staining of ER after presence of C36, indicating that C36 had no significant effect on cultivated H1299 cells, but C36 resulted in cytoplasmic vacuolization in H1563 cells. Accordingly, Fig. 5E, 5F, resp. Fig. 7E, 7F of the overlays of the microscopic pictures with the respective ER staining show ER stress induced vacuolisation.

Fig. 6 and Fig. 8 show results of qRT-PCR for expression levels of ER stress-related genes for H1563 and H1299 cell lines after 48h of culture with or without peptide C36. It was found that treatment with C36 in H1563 cells induced expression ATF6 (Activating Transcription Factor 6), ERN1 (Endoplasmic Reticulum Nucleus 1, also known as IRE1), ERO1A (ER Oxidoreductase 1 Alpha) and XBP1 (X-box binding protein 1). By contrast, expression of DDIT3 (DNA Damage-Inducible Transcript 3, also known as CHOP) decreased. Notably, CHOP is usually not upregulated in ferroptosis and is not considered a reliable marker for this form of cell death. CHOP is a pro-apoptotic transcription factor. Ferroptosis is an iron-dependent form of regulated necrosis, characterized by lipid peroxidation, mitochondrial shrinkage, and reactive oxygen species (ROS) accumulation, and does not rely on the classical ER stress which would indicate the pathway involving CHOP. The effect of C36 is that CHOP is even decreased in H1563 cells. Regarding H1299 cells, peptide C36 had no significant effect on ER stress and related gene expression.

Fig. 9A and Fig. 9B, resp. Fig. 10A and Fig. 10B show total cellular reactive oxygen species (ROS) levels as assessed by flow cytometry (FACS) using the fluorescent dye 2',7'-dichlorofluorescin diacetate (DCFDA) subsequent to 48 hours of treatment with the peptide C36. Cells cultured without peptide served as a negative control (Ctrl), and cells treated with tert-butyl hydroperoxide (TBHP) was used as a positive control. Upon oxidation, DCFDA emits green fluorescence, detected at 485/530 nm. In H1563 cells, peptide treatment resulted in a rightward shift of the fluorescence peak and increased mean fluorescence intensity, indicating elevated ROS levels. In contrast, H1299 cells showed no significant change in ROS following treatment by peptide C36.

Fig. 11 shows measurement results for lipid peroxidation, a key indicator of ferroptosis, as evaluated by flow cytometry using the ratio metric dye BODIPY^{™} 581/591 C11. Following oxidation of polyunsaturated lipids, the dye undergoes a fluorescence shift from red (emission ~590 nm) to green (emission ~510 nm). H1563 cells in medium alone (Ctrl) or pre-treated with C36 peptide (C36) for 48 hours were incubated for 30 min with the dye, and fluorescence was analyzed by flow cytometry. The extent of lipid peroxidation was quantified by calculating the ratio of green to red fluorescence, and the results were plotted using GraphPad Prism. An increase in the green/red fluorescence ratio indicates elevated lipid peroxidation, which is consistent with ferroptotic activity of C36. The results show that treatment of the cancer cells with C36 significantly increased lipid peroxidation.

Fig. 12 shows the FACS results for the mitochondrial membrane potential for H1563 cells that were cultured for 48 hours either alone or in the presence of peptide C36, using MitoTracker^{™} Deep Red, a dye that accumulates in mitochondria in a membrane potential-dependent manner. H1563 cells were treated with the peptide C36 and analyzed after 8 hours. An increase in fluorescence intensity was observed, indicating mitochondrial hyperpolarization at this time point. In contrast, the same analysis performed at 24 and 48 hours revealed no significant changes in mitochondrial membrane potential, suggesting that the hyperpolarization is a transient and early event following peptide C36 treatment.

Fig. 13 shows confocal microscopy pictures with staining for mitochondria with MitoTracker^{™} Red CMXRos (red, MtR, Fig. 13A, 13B), nuclei were counterstained with DAPI (blue, Fig. 13C, 13D). Microscopic evaluation revealed that peptide treatment led to mitochondrial fragmentation, as indicated by increased punctate mitochondrial structures, compared to the more elongated and tubular morphology observed in untreated control cells. FACS measurements for mitochondrial reactive oxygen species (ROS) levels using MitoSOX^{™} Red, a mitochondria-targeted dye that fluoresces upon oxidation by superoxide, PE staining as reference, are shown in Fig. 14A at 24 h, in Fig. 14B at 48 h after contact with C36. H1563 cells treated with the peptide C36 exhibited increased red fluorescence, indicating elevated mitochondrial ROS production. Antimycin A, a complex III inhibitor and known inducer of mitochondrial ROS, was used as a positive control to validate the assay.

Fig. 14C shows the data, with Antimycin A (AA) as a positive control. The mitochondrial ROS production in cells treated with peptide C36 was even higher than in the positive control with AA.

Fig. 15 shows confocal microscopy images of H1563 cells that were cultured for 48 hours either in medium alone (control, Fig. 15 A, 15C, 15E) or in the presence of the peptide C36 (C36, Fig. 15B, 15D, 15F) and analysed for iron accumulation. Increased intracellular ferrous iron (Fe²⁺) levels following peptide C36 treatment when stained with BioTracker^{™} 575 Red Fe²⁺ (red) to detect labile Fe²⁺, and nuclei were counterstained with DAPI (blue, Fig. 15 C and 15D). Peptide C36-treated cells displayed enhanced red fluorescence, indicating elevated Fe²⁺ accumulation.

Fig. 16 shows FACS results for intracellular Fe²⁺ using BioTracker^{™} 575 Red. Cells treated with peptide, Erastin (positive control), or a combination of C36+Erastin showed increased fluorescence intensity, reflecting elevated Fe²⁺ levels. Mean fluorescence intensity (MFI) values were quantified and plotted using GraphPad Prism.

Fig. 17 shows a Western blot analysis of transferrin receptor (TFRC) protein levels over time (0-48 hours) following peptide C36 treatment of cultivated H1563 cells. A time-dependent increase in TFRC expression is observed, consistent with enhanced iron uptake mechanisms.

Fig. 18 shows results of quantitative real-time PCR (qRT-PCR) analysis for the mRNA encoding TFRC. The increase of TFRC transcripts at 12 hours (Fig. 18A), 24 hours (Fig. 18B), and 48 hours (Fig. 18C) post-treatment confirms the results of the protein-level increase of TFRC (CD71) receptor expression shown in Fig. 17.

Fig. 19A, 19C show light microscopy images of H1299 cells cultured for 48 hours either in medium alone (Fig. 19A) or in the presence of the C36 peptide (Fig. 19C). Fig. 19B, 19D show light microscopy images of H1563 cells cultured for 48 hours either in medium alone (Fig. 19B) or in the presence of the C36 peptide (Fig. 19D). It was found that cells treated with the peptide C36 show prominent cytoplasmic vacuolization compared to untreated controls. This morphological change is indicative of a stress response or non-apoptotic cell death pathway and is consistent with ferroptosis or lysosomal dysfunction induced by C36.

Fig. 20A shows total genomic DNA was isolated by phenol:chloroform:isoamyl alcohol extraction from H1563 cells that were treated (C36) or untreated (Ctrl). DNA samples were separated on a 2% agarose gel in 0.5× TBE (Tris-borate- ethylenediaminetetraacetic acid EDTA) buffer for 3 hours, followed by post-staining with GelRed for 20 min. The gel image shows that cells treated with the peptide do not exhibit DNA fragmentation, as indicated by the absence of a characteristic DNA ladder pattern. In contrast, total DNA extracted from H1563 cells treated with staurosporine (STS, Fig. 20B), a known inducer of apoptosis, display clear DNA fragmentation, confirming assay sensitivity.

Fig. 21 shows results of quantitative real-time PCR (qRT-PCR) analysis for SQSTM1 (p62) expression levels in H1563 cells treated with the peptide C36. mRNA levels were measured at 12 hours (Fig. 21A), 24 hours (Fig. 21B), and 48 hours (Fig. 21C) after the treatment. The data show an increase in SQSTM1 expression at all time points, indicating activation of cellular stress responses such as autophagy or oxidative stress signalling. Expression levels were normalized to housekeeping gene controls (RNA Polymerase II Subunit A , POLAR2A).

### Example 2: In vitro killing of cancer cells by peptide C36 in combination with an apoptosis-inducing pharmaceutic

The combination of peptide C36 with an apoptosis-inducing pharmaceutical for use in treating cancer is shown on the example of cisplatin, a commonly used platinum-based chemotherapeutic agent. Contacting cancer cells peptide with C36 in combination with cisplatin showed an additive effect on cancer cell death. This suggests that C36 does not interfere with cisplatin's mechanism of action, e.g. effecting DNA crosslinking, but rather that C36 complements the activity of this exemplary apoptosis-inducing agent through distinct pathways such as oxidative stress induction, lysosomal damage, or ferroptosis-like cell death.

Figs. 22A-22G show the survival of different cultivated cells when contacted with medium containing C36 (C36), only cisplatin (Cisplatin), or a combination of C36 with cisplatin (Cisplatin+C36), in comparison to medium only (Ctrl).

These results show that the combination of C36 with an apoptosis-inducing agent, exemplified by cisplatin, is more effective in treatment of cancer than either C36 or the apoptosis-inducing agent alone.

### Example 3: In vitro killing of cancer cells by peptide C36 or C42 and combinations with an apoptosis-inducing pharmaceutic

The effect of peptide C36 on cancer cells was found to be dependent on expression of TFRC by cancer cells. On the example of cultivated H1299 cancer cells, analyses show that sensitivity to peptide C36 as well as, e.g. due to the different mode of action of C36, sensitivity to a combination of peptide C36 with an apoptosis-inducing pharmaceutical is higher for cells expressing high levels of TFRC.

Fig 23A for cultivation in medium alone, Fig. 23B for cultivation in medium containing peptide C36 depicts cell viability of H1299 cancer cells in relation to levels of mRNA encoding TFRC, standardized to POLR2A mRNA. This shows that high levels of TFRC expression in cancer cells increase the sensitivity to C36, resulting in lower cell viability.

Further, this shows that preferably, the cancer to be treated by use of the compound of the invention preferably is a cancer expressing TFRC at a higher level, e.g. at a higher level than non-cancerous cells.

As shown in Figs. 24 and 25, each of peptide C36 and peptide C42 alone or in combination with an apoptosis-inducing pharmaceutical are effective against cancer.

On the exemplary lung cancer cell lines H1563 and 2106T, each cultivated for 48 h in presence of C36 or C42, each at a constantly maintained concentration of 40µg/mL culture medium, and one of C36 and C42 in combination with etoposide (Eto), staurosporin (STS), or osimertinib (Osi), subsequent to 48 h culture activity against cancer cells was found.

For H1563, the proportion of viable cells are shown in Fig. 24A for medium alone (Control), for C36 alone (C36), C42 alone (C42 40µg), for C36 in combination with 15 µg/mL cisplatin (Cis 15µg+C36), and for cisplatin in combination with C42 (Cis 15µg+C42), showing higher anti-cancer activity for C36 and C42 than for cisplatin alone, and even higher activity for each combination of C36 or C42 with cisplatin.

Fig. 24B shows higher activity for C36 alone (C36), C42 alone (C42 40µg), for C36 in combination with 25 µM etoposide (Eto 25µM+C36), and for etoposide in combination with C42 (Eto 25µM+C42) than for etoposide alone (Eto 25µM).

Fig. 24C shows higher activity for C36 alone (C36), C42 alone (C42 40µg), for C36 in combination with 5 µM osimertinib (Osi 5µM+C36), and for osimertinib in combination with C42 (Osi 5µM+C42) than for osimertinib alone (Osi 5µM).

Fig. 24D shows higher activity for C36 alone (C36), C42 alone (C42 40µg), for C36 in combination with 5 µM staurosporin (STS 250nM+C36), and for staurosporin in combination with C42 (STS 250nM+C42) than for staurosporin alone (STS 250nM).

For 2106T, the proportion of viable cells are shown in Fig. 25A for medium alone (Control), for C36 alone (C36), C42 alone (C42 40µg), for C36 in combination with 15 µg/mL cisplatin (Cis 15µg+C36), and for cisplatin in combination with C42 (Cis 15µg+C42), showing a similar level of anti-cancer activity for C36 and C42 as for cisplatin alone, and for each combination of C36 or C42 with cisplatin.

Fig. 25B shows higher activity for C36 alone (C36), a similar activity of C42 alone (C42 40µg) and for etoposide in combination with C42 (Eto 25µM+C42) as for etoposide alone (Eto 25µM), but a higher activity for C36 in combination with 25 µM etoposide (Eto 25µM+C36).

Fig. 25C shows higher activity for C36 alone (C36) and even higher for C36 in combination with 5 µM osimertinib (Osi 5µM+C36) than for osimertinib alone (Osi 5µM), and a similar activity of C42 alone (C42 40µg) and for osimertinib in combination with C42 (Osi 5µM+C42) as for osimertinib alone (Osi 5µM).

Fig. 25D shows similar activity for C36 alone (C36), for C42 alone (C42 40µg), and for C36 in combination with 250nM staurosporin (STS 250nM+C36) as for staurosporin alone (STS 250nM), and a higher activity for staurosporin in combination with C42 (STS 250nM+C42) than for staurosporin alone (STS 250nM).

The effect of the compound of the invention to significantly enhance the efficacy of an apoptosis-inducing pharmaceutical is shown on the example of peptide C36 with cisplatin.

Fig. 26 shows the effect on C36 and combinations of C36 with different concentrations of cisplatin on viability of H1563 cancer cells after cultivation for 48 hours.

In detail, 40 µg/mL C36 alone (C36) had a higher effect on reducing cancer cells than 5 µg/mL cisplatin (Cis 5µg) or than 10 µg/mL cisplatin (Cis 10µg), and a similar effect as 15 µg/mL cisplatin (Cis 15µg). Higher activities were found for the combination of C36 with 5 µg/mL cisplatin (Cis 5µg+C36), even higher for the combination of C36 with 10 µg/mL cisplatin (Cis 10µg+C36), and still higher for the combination of C36 with 15 µg/mL cisplatin (Cis 15µg+C36).

Fig. 27A shows viability of H1299 cancer cells at 90%, at 60%, and at 30% confluency in presence of medium only (Control), in medium with added peptide C36 (C36), in medium with added Cisplatin (Cis), and in medium with added peptide in combination with Cisplatin (C36+Cis). This shows that in the in vitro culture, these cancer cells are sensitive to peptide C36 at low confluency, but highly sensitive to peptide C36 in combination with the exemplary apoptosis-inducing pharmaceutical.

Fig. 27B depicts expression levels of TFRC in H1299 cancer cells at 90%, at 60%, and at 30% confluency in presence of medium only (Control), in medium with added peptide C36 (C36), in medium with added Cisplatin (Cis), and in medium with added peptide in combination with Cisplatin (C36+Cis), showing that in the in vitro culture at cell densities less than confluency, peptide C36 results in a higher expression of TFRC compared to medium alone, whereas Cisplatin alone or in combination with peptide C36 has little to no effect of TFRC expression.

## Claims

1. Compound for use in the treatment of cancer, **characterized in that** the compound is a peptide consisting of amino acids No. 1-42, 2-42, 3-42, 4-42, 5-42, 6-42 and/or 7-42 of amino acid sequence LEAIPMSIPPEVKFNKPFVFLMIEQNTKSPLFMG KVVNPTQK (SEQ ID NO: 1).

2. Compound for use in the treatment of cancer according to claim 1, **characterized in that** the peptide consists of the amino acid sequence of SEQ ID NO: 2.

3. Compound for use in the treatment of cancer according to one of the preceding claims, **characterized in that** the compound is used in combination with an apoptosis-inducing pharmaceutical.

4. Compound for use in the treatment of cancer according to one of the preceding claims, **characterized in that** the cancer has an expression of TfR1 higher by at least 30%, by at least 50% or by at least 100% as determined as expression of the TFRC gene than non-cancerous cells of the same type of tissue.

5. Compound for use in the treatment of cancer according to one of the preceding claims, **characterized in that** the cancer is susceptible to an apoptosis-inducing pharmaceutical.

6. Compound for use in the treatment of cancer according to one of the preceding claims, **characterized in that** the cancer is refractory to treatment by an apoptosis-inducing pharmaceutical.

7. Compound for use in the treatment of cancer according to one of the preceding claims, **characterized in that** the treatment is subsequent to cancer treatment by an apoptosis-inducing pharmaceutical.

8. Compound for use in the treatment of cancer according to one of the preceding claims, **characterized in that** the cancer is one of non-small cell lung cancer (NSCLC), triple-negative breast cancer (TNBC), hepatocellular carcinoma, and glioblastoma.

9. Compound for use in the treatment of cancer according to one of claims 3 to 8, **characterized in that** the apoptosis-inducing pharmaceutical is Cisplatin, Adiposide, Etoposide, Staurosporin, Osimertinib, or a combination of at least two of these.

10. Compound for use in the treatment of cancer according to one of claims 3 to 9, **characterized in that** the apoptosis-inducing pharmaceutical is administered to a reduced dosage, e.g. to 70%, 60%, 50% 40% or 30% of the dosage for the apoptosis-inducing pharmaceutical when used as a single active agent, or as a combination of at least two apoptosis-inducing pharmaceuticals in the treatment of cancer.

11. Compound for use in the treatment of cancer according to one of the preceding claims, **characterized in that** the peptide is coupled to a nanoparticle.

12. Compound for use in the treatment of cancer according to one of claims 3 to 11, **characterized in that** the peptide consisting of amino acids No. 1-42, 2-42, 3-42, 4-42, 5-42, 6-42 and/or 7-42 of amino acid sequence SEQ ID NO: 1 is for administration concurrent with administration of an apoptosis-inducing pharmaceutical, for administration prior to administration of an apoptosis-inducing pharmaceutical, or for administration subsequent to administration of an apoptosis-inducing pharmaceutical.
